Europäisches Patentamt

European Patent Office

Office européen des brevets

(11) Numéro de publication : **0 341 124 B1**

(12) **FASCICULE DE BREVET EUROPEEN**

(45) Date de publication du fascicule du brevet :
**18.03.92 Bulletin 92/12**

(51) Int. Cl.⁵ : **C07C 45/00, C07C 45/45, C07C 47/14, C07C 49/167**

(21) Numéro de dépôt : **89401138.6**

(22) Date de dépôt : **21.04.89**

---

(54) **Procédé de préparation de dérivés fluorés fonctionnels.**

---

Le dossier contient des informations techniques présentées postérieurement au dépôt de la demande et ne figurant pas dans le présent fascicule.

(30) Priorité : **04.05.88 FR 8806000**

(43) Date de publication de la demande :
**08.11.89 Bulletin 89/45**

(45) Mention de la délivrance du brevet :
**18.03.92 Bulletin 92/12**

(84) Etats contractants désignés :
**BE CH DE ES FR GB IT LI NL**

(56) Documents cités :
**EP-A- 0 254 652**
**FR-A- 2 521 987**
**US-A- 4 484 993**

(73) Titulaire : **ATOCHEM**
**4 & 8, Cours Michelet La Défense 10**
**F-92800 Puteaux (FR)**

(72) Inventeur : **Benefice-Malouet, Sylvie**
**Résidence Le Prieuré**
**Allée P 1 31 bis, Avenue Saint-Lazarre**
**F-34000 Montpellier (FR)**
Inventeur : **Blancou, Hubert Les Terrasses du Peyrou**
**Bâtiment 6 781 Avenue Monsieur Teste**
**F-34100 Montpellier (FR)**
Inventeur : **Commeyras, Auguste**
**2 Impasse des Ecoles**
**F-34170 Clapiers (FR)**

(74) Mandataire : **Leboulenger, Jean et al**
**ATOCHEM Département Propriété Industrielle**
**F-92091 Paris la Défense 10 Cédex 42 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne le domaine des dérivés fluorés fonctionnels (aldéhydes, cétones, amides) et a plus particulièrement pour objet la synthèse des aldéhydes perfluorés du type $R_FCHO$ et de leurs hydrates $R_FCH(OH)_2$ où $R_F$ désigne un radical perfluoroalkyle linéaire ou ramifié.

Ces aldéhydes perfluorés sont utiles comme matières premières pour la synthèse de produits chimiques à chaîne perfluorée utilisables, par exemple, comme agent de surface. Ils peuvent notamment être réduits en alcools polyfluorés $R_F\text{-}CH_2OH$.

Parmi les diverses voies d'accès à ces aldéhydes perfluorés connues à ce jour, on peut mentionner plus particulièrement les suivantes :

1) L'ozonolyse de composés du type $CF_3(CF_2)_nCH = CHR$ qui permet l'obtention directe d'aldéhydes du type $CF_3(CF_2)_nCHO$ avec un rendement de 25 % (brevets DE 2 556 844 et GB 1 473 807).

2) La réaction du perfluorobutyronitrile avec un magnésien tel que le bromure d'isopropylmagnésium conduisant à la formation de 37 % de perfluorobutyraldéhyde (Mc Bee et al., J. Am. Chem. Soc., 1955, 77, 917).

3) La réaction du tétrafluoroéthylène avec $(CF_3)_3C\text{-}CH(NMe_2)_2$ conduisant après hydrolyse à l'aldéhyde $(CF_3)_3C\text{-}CF_2\text{-}CF_2\text{-}CHO$ (Zeifman et al., Dokl. Akad. Nauk., SSSR, 1982, 265, 2, 347).

On peut également signaler l'obtention d'aldéhydes perfluorés acétyléniques $R_FC{\equiv}C\text{-}CHO$ avec un rendement compris entre 15 et 40 % par réaction de Wittig entre un chlorure d'acide perfluoré $R_FCOCl$ et un ylure de phosphore du type $(C_6H_5)_3P = CH\text{-}CHO$, suivie d'une pyrolyse à 220°C sous 1333 Pa (Shen Yanchang et al., Synthesis, 1985, 2, 159), ainsi que l'obtention du perfluorobenzaldéhyde avec un rendement de 40 % par réaction du perfluorophényllithium avec le N-méthylformamide (Coe et al., J. Chem. Soc., 1962, 3227) ou avec un rendement de 62,4 % par réaction du chlorure de perfluorophénylmagnésium avec le N-méthylformanilide (Vorozhtsov et al., Dokl. Akad. Naut., SSSR, 1964, 159, 125 et CA, 1965, 62, 4045a).

On sait par ailleurs que les iodures de perfluoroalkyle $R_FI$ réagissent dans le diméthylsulfoxyde ou le diméthylformamide avec le couple métallique zinc-cuivre et des substrats tels que $SO_2$ ou $CO_2$ pour conduire aux sulfonates et carboxylates de zinc, précurseurs des acides perfluorés correspondants ( brevets FR 2 342 950, 2 373 503 et 2 374 287) et que les iodures polyfluorés du type $R_FCH_2CH_2I$ réagissent avec le couple zinc-cuivre dans le diméthylsulfoxyde ou le diméthylformamide pour produire, après oxydation par l'oxygène gazeux et hydrolyse, les alcools fluorés $R_FCH_2CH_2OH$ (brevet FR 2 521 987). Ces réactions connues ont été effectuées en l'absence d'initiateur radicalaire.

Il a maintenant été trouvé qu'on peut accéder facilement aux aldéhydes perfluorés $R_FCHO$ et à leurs hydrates $R_FCH(OH)_2$, ainsi qu'à des cétones et amides fluorés comme indiqué dans les revendications, en faisant réagir un iodure de perfluoroalkyle $R_FI$ sur le couple métallique zinc-cuivre avec un amide N,N-dialkylé utilisé comme solvant, en présence d'un initiateur radicalaire.

Le procédé selon l'invention s'applique plus particulièrement aux iodures de perfluoroalkyle dont le radical $R_F$, linéaire ou ramifié, contient de 2 à 20 atomes de carbone et, de préférence, de 4 à 12 atomes de carbone.

Le couple métallique zinc-cuivre à utiliser pour la mise en oeuvre du procédé selon l'invention peut être obtenu, par exemple, par addition de zinc en poudre à une solution bouillante d'acétate de cuivre dans l'acide acétique, puis lavage du solide à l'acide acétique et séchage. D'autres sels de cuivre que l'acétate peuvent être utilisés, par exemple le chlorure ou le sulfate en milieu acide chlorhydrique-eau. Le rapport molaire Zn/Cu peut varier de 30 à 150, mais il est de préférence compris entre 50 et 120. La quantité de zinc sous forme de couple Zn/Cu à utiliser par mole de $R_FI$ peut varier de 1 à 2 moles et est de préférence comprise entre 1 et 1,5 mole.

Comme amide N,N-dialkylé qui sert en même temps de réactif et de solvant, on utilise de préférence le diméthylformamide, mais on peut généralement employer les composés $RCONR_1R_2$, R désignant un atome d'hydrogène ou R' qui est un radical méthyle ou éthyle, $R_1$ et $R_2$ représentant chacun un radical alkyle en $C_1$ à $C_4$. La quantité d'amide N,N-dialkylé à mettre en oeuvre peut varier de 1,5 à 5 moles par mole de $R_FI$ et est de préférence comprise entre 1,8 et 2,2 moles.

L'initiateur radicalaire préféré est l'azobisisobutyronitrile, mais on peut généralement employer tout initiateur, par exemple, l'acide 4,4'-azobis(4-cyanovalérique), le chloroformiate d'éthyle. La quantité d'initiateur a mettre en oeuvre peut varier de 0, 01 à 0,1 mole par mole de $R_FI$ et est de préférence comprise entre 0, 02 et 0, 04 mole.

La réaction s'effectue bien a une température allant de -20°C à +25°C. Elle est de préférence réalisée à environ 0°C en ajoutant rapidement le iodure de perfluoroalkyle à une dispersion du couple Zn/Cu dans l'amide N,N-dialkylé contenant l'initiateur radicalaire. La réaction étant exothermique, il est généralement utile de refroidir le mélange réactionnel par tout moyen approprié.

Le ou les dérivés fluorés fonctionnels formés peuvent être isolés de façon connue en soi, par exemple, filtration, décantation, distillation sous vide, sublimation. Lorsque l'amide N,N-dialkylé est du type formamide, la distillation du mélange réactionnel filtré fournit généralement un mélange de l'aldéhyde per-

fluoré $R_FCHO$ et du N,N-dialkyl-formamide. Par traitement de ce mélange avec de l'eau, on forme l'hydrate $R_FCH(OH)_2$ qui peut être isolé par filtration et facilement purifié par sublimation ; sa déshydratation par distillation sur $P_2O_5$ permet d'obtenir l'aldéhyde $R_FCHO$ pur. Comme le montrent les exemples 4 et 6, un rapport molaire N,N-dialkyl-formamide/$R_FI$ élevé conduit à la formation concomitante des amides fluorés $R_FCONR_1R_2$ correspondants.

Lorsqu'au lieu d'un N,N-dialkyl-formamide on met en oeuvre un amide $R'CONR_1R_2$ où $R'$ est un radical méthyle ou éthyle, le procédé selon l'invention conduit aux amides fluorés précités et aux cétones fluorées $R_FCOR'$ correspondantes.

Les exemples suivants illustrent l'invention sans la limiter.

## EXEMPLE 1 : SYNTHESE DU PERFLUOROHEPTANAL

### a) Préparation du couple métallique Zn/Cu

A une solution de 0,2 g d'acétate de cuivre (0,001 mole) dans 10 ml d'acide acétique porté à ébullition, on ajoute par petites fractions 6,5 g de zinc (0,1 mole) en poudre. Après refroidissement, le produit est lavé plusieurs fois a l'acide acétique, puis on récupère le couple métallique par décantation et on élimine l'acide acétique par évaporation sous pression réduite.

### b) Préparation de $C_6F_{13}CHO$

Le couple Zn/Cu fraîchement préparé est alors dispersé dans 15 ml de diméthylformamide, puis on ajoute 3 millimoles d'azobisisobutyronitrile. On introduit alors rapidement (environ 8 minutes) sous agitation, 44,6 g (0,1 mole) d'iodure de perfluorohexyle, tout en maintenant la température du mélange réactionnel à environ 0°C au moyen d'un bain d'alcool froid ou de glace.

La partie organofluorée est ensuite extraite par décantation, puis filtrée. Par distillation du filtrat sous pression réduite (133 Pa), on obtient 34 g d'un mélange contenant en mole 70 % de perfluoroheptanal $C_6F_{13}CHO$ (soit 31,3 g) et 30 % de diméthylformamide (soit 2,7 g), ce qui correspond à un rendement de 90 % en perfluoroheptanal par rapport au iodure de perfluorohexyle de départ. Les pourcentages molaires indiqués sont déterminés par intégration des signaux observés par RMN [1]H.

On obtient le même résultat si on remplace l'azobisisobutyronitrile par la même quantité molaire d'acide 4,4'-azobis(4-cyanovalérique).

Le perfluoroheptanal est obtenu, exempt de diméthylformamide, sous sa forme hydratée $C_6F_{13}CH(OH)_2$ par sublimation.

## EXEMPLE 2 : SYNTHESE DU PERFLUOROPENTANAL

On opère comme à l'exemple 1, mais avec 34,6 g (0,1 mole) d'iodure de perfluorobutyle $C_4F_9I$. On obtient 26,5 g d'un mélange contenant en moles 70 % de perfluoropentanal $C_4F_9CHO$ (soit 23,55 g) et 30 % de diméthylformamide (soit 2,95 g), ce qui correspond à un rendement de 95 % en perfluoropentanal par rapport à l'iodure de perfluorobutyle de départ.

## EXEMPLE 3 : SYNTHESE DU PERFLUORONONANAL

Si on répète l'exemple 1 avec 54,6 g (0,1 mole) d'iodure de perfluorooctyle $C_8F_{17}I$, on obtient 40,7 g d'un mélange contenant en mole 70 % de perfluorononanal $C_8F_{17}CHO$ (soit 38,05 g) et 30 % de diméthylformamide (soit 2,65 g), ce qui correspond à un rendement de 85 % de perfluorononanal par rapport à l'iodure de perfluorooctyle de départ.

## EXEMPLE 4

On opère comme à l'exemple 1, mais avec 30 ml de diméthylformamide. Après réaction, le mélange réactionnel comprend deux phases qu'on sépare par décantation.

La phase inférieure est traitée comme à l'exemple 1. On obtient 26,5 g d'un mélange contenant en moles 70 % de perfluoroheptanal (soit 24,3 g) et 30 % de diméthylformamide (soit 2,2 g), ce qui correspond à un rendement de 70 % en perfluoroheptanal par rapport à l'iodure de perfluorohexyle de départ.

La phase supérieure, distillée sous pression réduite et chromatographiée, fournit 7,8 g de l'amide $C_6F_{13}CON(CH_3)_2$.

## EXEMPLE 5

On répète l'exemple 4, mais en remplaçant le diméthylformamide par 35 ml de diméthylacétamide. On obtient alors 16,3 g de méthyl perfluorohexyl cétone $C_6F_{13}COCH_3$ et 17,6 g de l'amide $C_6F_{13}CON(CH_3)_2$.

## EXEMPLE 6

On répète l'exemple 4, mais en remplaçant le diméthylformamide par 40 ml de diéthylformamide. On obtient alors :
– 24,3 g d'un mélange contenant en moles 80 % de perfluoroheptanal (soit 22,65 g) et 20 % de diéthylformamide (soit 1,65 g), ce qui correspond à un rendement de 65 % par rapport à l'iodure de perfluorohexyle de départ ;
– 10,9 g de l'amide $C_6F_{13}CON(C_2H_5)_2$.

## EXEMPLE 7

On opère comme à l'exemple 1-b, mais en opérant à la température ambiante (environ 20°C). Après purification, on obtient 30,3 g d'un mélange contenant en moles 70 % de perfluoroheptanal (soit 27,8 g) et 30 % de diméthylformamide (soit 2,5 g), ce qui correspond à un rendement de 80 % en perfluoroheptanal par rapport à l'iodure de perfluorohexyle engagé.

## EXEMPLE 8

On opère comme à l'exemple 1-b, mais en remplaçant l'azobisisobutyronitrile par 1 g de chloroformiate d'éthyle. On obtient alors 24,6 g d'un mélange contenant en moles 70 % de perfluoroheptanal (soit 22,6 g) et 30 % de diméthylformamide (soit 2 g), ce qui correspond à un rendement de 65 %.

## Revendications

1. Procédé de préparation d'aldéhydes perfluorés $R_FCHO$, de cétones fluorées $R_FCOR'$ et/ou d'amides fluorés $R_FCONR_1R_2$ dans lesquels $R_F$ représente un radical perfluoroalkyle, linéaire ou ramifié, contenant de 2 à 20 atomes de carbone, R' représente un radical méthyle ou éthyle, $R_1$ et $R_2$ représentent chacun un radical alkyle en $C_1$ à $C_4$, caractérisé en ce que l'on fait réagir, en présence d'un initiateur radicalaire, un iodure de perfluoroalkyle $R_FI$ sur le couple métallique zinc-cuivre avec un amide $RCONR_1R_2$, où R désigne un atome d'hydrogène ou R'.

2. Procédé selon la revendication 1 pour la préparation des aldéhydes perfluorés $R_FCHO$, caractérisé en ce que l'amide $RCONR_1R_2$ est un N,N-dialkylformamide, de préférence le diméthylformamide.

3. Procédé selon la revendication 1 ou 2, dans lequel l'initiateur radicalaire est l'azobisisobutyronitrile ou l'acide 4,4'-azobis(4-cyanovalérique).

4. Procédé selon l'une des revendications 1 à 3, dans lequel on opère à une température allant de -20°C à +25°C, de préférence à environ 0°C.

5. Procédé selon l'une des revendications 1 à 4, dans lequel le radical perfluoroalkyle $R_F$ contient de 4 à 12 atomes de carbone.

6. Procédé selon l'une des revendications 1 à 5, dans lequel on utilise de 1 à 2 moles de zinc sous forme de couple Zn/Cu, de 1,5 à 5 moles d'amide $RCONR_1R_2$ et de 0,01 à 0,1 mole d'initiateur radicalaire, par mole d'iodure de perfluoroalkyle.

7. Procédé selon l'une des revendications 1 à 6, dans lequel le rapport molaire Zn/Cu est compris entre 30 et 150 et, de préférence, compris entre 50 et 120.

## Patentansprüche

1. Verfahren zur Herstellung von perfluorierten Aldehyden $R_FCHO$, von fluorierten Ketonen $R_FCOR'$ und/oder von fluorierten Amiden $R_FCONR_1R_2$, in denen $R_F$ ein Perfluoralkylrest, geradkettig oder verzweigt, der 2 bis 20 Kohlenstoffatome enthält, bedeutet, R' einen Methyl- oder Ethylrest bedeutet, $R_1$ und $R_2$ jeweils einen $C_1$-$C_4$ Alkylrest bedeuten, dadurch gekennzeichnet, daß man in Gegenwart eines radikalischen Starters ein Perfluoralkyljodid $R_FI$ über dem Metallpaar Zink/Kupfer mit einem Amid $RCONR_1R_2$, worin R ein Wasserstoffatom oder R' bezeichnet, reagieren läßt.

2. Verfahren nach Anspruch 1 zur Herstellung von perfluorierten Aldehyden $R_FCHO$, dadurch gekennzeichnet, daß das Amid $RCONR_1R_2$ ein N,N-Dialkylformamid, vorzugsweise Dimethylformamid, ist.

3. Verfahren nach einem der Ansprüche 1 oder 2, in dem der radikalische Starter Azobisisobutyronitril oder 4,4'-Azobis(4-cyanovalerian)-säure ist.

4. Verfahren nach einem der Ansprüche 1 bis 3, in dem man bei einer Temperatur von -20°C bis +25°C, vorzugsweise etwa bei 0°C arbeitet.

5. Verfahren nach einem der Ansprüche 1 bis 4, in dem der Perfluoralkylrest $R_F$ 4 bis 12 Kohlenstoffatome enthält.

6. Verfahren nach einem der Ansprüche 1 bis 5, in dem man 1 bis 2 mol Zink unter Bildung des Zn/Cu-Paares, 1,5 bis 5 mol des Amids $RCONR_1R_2$ und 0,01 bis 0,1 mol des radikalischen Starters pro mol Perfluoralkyljodid verwendet.

7. Verfahren nach einem der Ansprüche 1 bis 6, in dem das Molverhältnis Zn/Cu zwischen 30 und 150 und vorzugsweise zwischen 50 und 120 liegt.

## Claims

1. Process for the preparation of perfluoroaldehydes $R_FCHO$, of fluoroketones $R_FCOR'$ and/or of fluoroamides $R_FCONR_1R_2$ in which formulae $R_F$ denotes a linear or branched perfluoroalkyl radical containing from 2 to 20 carbon atoms, R' denotes a methyl or ethyl radical, and each of $R_1$ and $R_2$ denotes a $C_1$-$C_4$ alkyl radical, characterised in that a perfluoroalkyl iodide $R_FI$ is reacted, in the presence of a radical initiator, over the zinc-copper metal couple with an amide $RCONR_1R_2$, where R denotes a hydrogen atom or R'.

2. Process according to Claim 1 for the preparation of perfluoroaldehydes $R_FCHO$, characterised in that the amide $RCONR_1R_2$ is an N, N-dialkylformamide, preferably dimethylformamide.

3. Process according to Claim 1 or 2, in which the radical initiator is azobisisobutyronitrile or 4,4'-azobis(4-cyanovaleric) acid.

4. Process according to one of Claims 1 to 3, in which the operation is carried out at a temperature ranging from -20°C to +25°C, preferably at approximately 0°C.

5. Process according to one of Claims 1 to 4, in which the perfluoroalkyl radical $R_F$ contains from 4 to 12 carbon atoms.

6. Process according to one of Claims 1 to 5, in which from 1 to 2 moles of zinc in the form of a Zn/Cu couple, from 1.5 to 5 moles of amide $RCONR_1R_2$ and from 0.01 to 0.1 mole of radical initiator are employed per mole of perfluoroalkyl iodide.

7. Process according to one of Claims 1 to 6, in which the Zn/Cu molar ratio is between 30 and 150 and, preferably, between 50 and 120.